# EUROPEAN PATENT APPLICATION

(11) **EP 2 868 665 A1**
(43) Date of publication of application: **06.05.2015**
(21) Application number: 14757125.1
(22) Date of filing: 27.02.2014
(51) Int. Cl.: C07K 7/08, C07K 16/06, C07K 16/10, A61K 39/42, A61P 31/14

(54) **HEPATITIS C VIRUS B CELL EPITOPE PEPTIDE PUHI26 AND APPLICATIONS THEREOF**

(30) Priority: 27.02.2013 CN 201310062069
(71) Applicant: Peking University People's Hospital, Beijing 100044 (CN)
(72) Inventor: WEI, Lai, Beijing 100044 (CN); LIU, Ruyu, Beijing 100044 (CN)
(74) Representative: Kutzenberger Wolff & Partner
(86) International application number: PCT/CN2014/072645
(87) International publication number: WO 2014/131362

(57) **Abstract**

Provided is a hepatitis C virus B-cell epitope peptide PUHI26, where the amino acid sequence thereof is THKFNSSGCPERMASCRPID. Also provided is a protective antibody capable of neutralizing hepatitis C virus 1a, 1b, 3a, 4a, 5 and 6 geno-type/subtype HCVpp.

## Description

### Technical Field

The present invention relates to the field of immunology, in particular, relates to a hepatitis C virus B-cell epitope peptide PUHI26 and application thereof.

### Background Art

Hepatitis C virus (HCV) is single-stranded positive-strand RNA virus, belonging to the *Flaviviridae* family. HCV genome comprises a single long open reading frame encoding a polyprotein consisting of about 3,000 amino acid residues. The polyprotein is cleaved into three main structural proteins and several non-structural proteins essential for virus replication under the action of cells and viral protease.

HCV genome is highly mutated, and only 60% of nucleotide sequences among the highly mutated isolate strains are homologous. Currently, HCV can be divided into 6 genotypes and different subtypes. In China, HCV genotypes 1b and 2a are common, wherein HCV genotype 1b is dominant. In some regions, HCV genotypes 1a, 2b and 3b have been reported, and HCV genotype 6 is mainly found in Hongkong and Macao as well as in some border provinces of South.

At present, about 180 million people around the world are infected with HCV, wherein 80% of infected people would be chronic, and then develop to hepatic cirrhosis and hepatocellular carcinoma. In China, there are about 6.5 million chronically HCV-infected people. As for the HCV chronic infection, the main treatment manner is a standard treatment with interferon plus ribavirin, however, it has poor therapeutic effect against genotypes 1 and 4, moreover, due to obvious toxic and adverse effect and high cost for treatment, a large number of HCV-infected people are forced to abandon the treatment. The HCV infection has become the main reason for liver transplantation operation in western countries, which is also one of important reasons in our country. Moreover, the standard treatment regimen has poor therapeutic effect in blocking the reinfection of transplanted liver caused by HCV infection, thus a new prevention and treatment regimen is in urgent need.

So far, more protective B-cell epitopes against HCV envelope proteins, e.g. epitopes 313-327, 396-407, 412-423 and 613-621, etc., have been identified, and the corresponding antibodies of such epitopes have good neutralization activity against HCV. One protective linear B-cell epitope peptide 412-423 (QLINTNGSWHIN), of which the corresponding antibody is named HCV1, has been studied mostly, and the phase II clinical trial thereof has been completed. In a chimpanzee animal model, 250 mg/kg HCV1 can completely block the infection of HCV representive strain H77 (genosubtype 1a), which indicates that in the field of liver transplantation caused by HCV infection, the antibody has wide application prospect in the aspect of blocking the HCV reinfection of transplanted liver, and alsoin the aspect of developing HCV vaccine. A series of experiments show that the identification of protective epitopes has very wide application prospect in both prevention and treatment fields of HCV infection.

### Summary of the Invention

The purpose of the present invention is to provide a novel hepatitis C virus B-cell epitope peptide PUHI26 and application thereof.

In order to achieve the purpose, the present invention provides a hepatitis C virus B-cell epitope peptide PUHI26, where the amino acid composition thereof is THKFNSSGCPERMASCRPID.

The present invention performs sequence comparison on the reported Chinese genosubtype 1b HCV isolated strain envelope protein sequences, and finds out the consensus sequence of full-length envelope protein. The full-length HCV envelope protein (consensus sequence), i.e. from the amino acids at position 192-746 (using hepatitis C virus H77 as reference standard, Accession No. NC_004102), can be divided into 57 different polypeptides (not including transmembrane domain) by employing overlapping peptide method, wherein in the sequences of 192-35, 384-413, 444-523, 544-613 and 624-717, each polypeptide has a length of 20 amino acids, and the adjacent polypeptides have 10 overlapped amino acids; and in the sequences of 404-453, 514-553 and 604-633, each polypeptide has a length of 15 amino acids, and the adjacent polypeptides have 10 overlapped amino acids. Such polypeptides are synthesized artificially, and used to immunize Balb/c mice with 50 µg/time for three times at an interval of 2 weeks, so as to obtain polyclonal antibody (antiserum). The polyclonal antibody obtained is subjected to titer determination, and the method comprises indirect ELISA (reference: Ndongo N, Berthillon P, Pradat P, Vieux C, Bordes 1, Berby F, Maynard M, Zoulim F, Trepo C, Petit MA. Hepatology 2010; 52:1531-1542). The serum is diluted into 8 concentrations with the ratios of 1:1,000, 1:2,000, 1:4,000, 1:8,000, 1:16,000, 1:32,000, 1:64,000 and 1:128,000. At the dilution ratio of 1:128,000, the OD value at 490 nm is ≥0.25, and the ratio of the OD value of polyclonal antibody to that of negative control is ≥2at the dilution ratio of 1:128,000, judging that the titer of the polyclonal antibody is qualified. The HCV polyclonal antibody obtained is subjected to HCV-pseudotyped particles (HCVpp) neutralization experiment, and the method comprises packaging 7 different geno-types/subtypes (1a, 1b, 2a, 3a, 4a, 5 and 6) of HCVpp having luciferase reporter gene (reference: Tong Y, Zhu Y, Xia X, Liu Y, Feng Y, Hua X, Chen Z, Ding H, Gao L, Wang Y, Feitelson MA, Zhao P, Qi ZT . J Virol, 2011; 85:2793-2802), mixing 57 HCV polyclonal antibodies each diluted at different ratios with the 7 different geno-types/subtypes of HCVpp respectively, then adding into a 96-wellplate pre-inoculated with Huh7.5 cells, detecting luciferase activity after 72 h, and comparing with the control to determine the HCVpp-neutralizing activities of different polyclonal antibodies (reference: Tarr AW, Urbanowicz RA, Jayaraj D, Brown RJ, McKeating JA, Irving WL, Ball JK. J Viro12012; 86:2739-2749), and thereby determine the protective B-cell epitope peptide.

The experimental results showed that the sequence of 444-463 (using hepatitis C virus H77 as reference standard, Accession No. NC_004102) is protective B-cell epitope peptide named PUHI26, where the corresponding amino acid sequence thereof is THKFNSSGCPERMASCRPID, and the corresponding polyclonal antibody thereof can neutralize geno-types/subtypes 1a, 1b, 3a, 4a, 5 and 6 of HCVpp.

The present invention also provides the use of the hepatitis C virus B-cell epitope peptide PUHI26 in the preparation of anti-HCV drugs. A polyclonal antibody is prepared by immunizing experimental animals (such as Balb/c mice) using the PUHI26 epitope peptide as immunogen together with an adjuvant. Alternatively, a humanized monoclonal antibody identifying PUHI26 epitope peptide antigen is prepared by immunizing experimental animals using the PUHI26 epitope peptide as immunogen together with adjuvant, and adopting hybridoma technology and recombinant DNA technology. The above polyclonal antibody or monoclonal antibody prepared is used separately or in combination to block HCV reinfection during and after liver transplantation operation in a chronic subject infected with HCV genotype 1b.

The present invention further provides a neutralizing antibody against HCV geno-types/subtypes 1a, 1b, 3a, 4a, 5 and 6, which is prepared by immunizing experimental animals (such as Balb/c mice) using hepatitis C virus B-cell epitope peptide PUHI26 as immunogen together with an adjuvant.

The aforementioned neutralizing antibody is prepared by coupling the epitope peptide PUHI26 with carrier proteins KLH and BSA respectively (wherein the BSA-coupled epitope peptide is used for serum antibody titer determination of Balb/c mice after the completion of immunizing) (reference: Darwish IA, Alzoman NZ, Abuhejail RM, El-Samani TE. Chem Cent J 2012; 6:125), mixing the KLH-coupled epitope peptide antigen with an adjuvant in equal volume, immunizing Balb/c mice via subcutaneous injection for 3 times after emulsified, and collecting the antibodies in the serum after the completion of immunizing.

In particular, it is prepared by coupling the epitope peptide PUHI26 with carrier proteins KLH and BSA respectively (wherein the BSA-coupled epitope peptide is used for serum antibody titer determination of Balb/c mice after the completion of immunizing), mixing the KLH-coupled epitope peptide antigen with an adjuvant in equal volume, after emulsified completely, immunizing 8- to 12-week-old Balb/c mice via subcutaneous injection at multiple sites on neck and back for 3 times on day 1, day 15 and day 29 respectively (Freund's complete adjuvant is used for the first time, and Freund's incomplete adjuvant is used for the second and third time. each mouse is injected with 500 µl of emulsified antigen each time, and the antigen dosage is 50 µg), detecting the serum antibody titer via indirect ELISA method after the completion of immunizing, and collecting the antibody in serum.

Through the overlapping peptide method, the present invention finds a novel linear protective B-cell epitope peptide of hepatitis C virus envelope protein, and obtains protective antibodies capable of neutralizing hepatitis C virus geno-types/subtypes 1a, 1b, 3a, 4a, 5 and 6 of HCVpp, where the amino acid sequence thereof is THKFNSSGCPERMASCRPID at positions 444-463 (using hepatitis C virus H77 as reference standard, Accession No. NC_004102). The determination of the protective B-cell epitope peptide provides a new solution for the development of therapeutic antibody and prophylactic vaccine for HCV.

### Brief Description of the Figures

Figure 1 is a flow chart showing a process for screening protective B-cell epitope peptide of HCV envelope protein according to Example 1 of the present invention.
Figure 2 shows titer determination results of corresponding antibodies of the protective B-cell epitope peptide at position 444-463 (PUHI26) according to Example 2 of the present invention.
Figures 3A-3F show the experimental results of neutralizing geno-types/subtypes 1a, 1b, 3a, 4a, 5 and 6 of HCVpp-for the corresponding antibodies of the protective B-cell epitope peptide at position 444-463 (PUHI26) according to Example 3 of the present invention.

### Detailed Embodiments of The invention

The following examples are used for describing the present invention, but not limiting the scope of the present invention. Unless especially indicated, the technical means used in Examples are conventional means well-known by a skilled person in the art, and all raw materials used are commercially available.

### Example 1 Acquisition of hepatitis C virus B-cell epitope peptide PUHI26

### 1.1 Preparation of polypeptide antigen and polyclonal antibody

The reported data about HCV full length protein sequence of Chinese HCV genosubtype 1b isolated strain are searched in NCBI database, and the collected sequences are subjected to comparison to find out the consensus sequence of full length envelope protein (using hepatitis C virus H77 as reference standard, Accession No. NC_004102, the amino acid residues are at positions 192-747). Starting from amino acid residue at 192, polypeptides with a length of 20 amino acids are artificially synthesized, and the adjacent polypeptides have 10 overlapped amino acids (e.g. if the first sequence is at 192-211, then the second one is at 202-221, and so on). The sequences at 404-453, 514-553 and 604-633 comprise possible CD81 (one of receptors for HCV when invading cells) binding sites, each synthesized polypeptide in such sequence ranges has a length of 15 amino acids, and the adjacent polypeptides have 10 overlapped amino acids. The transmembrane domain sequence of the envelope protein is not within the range of the synthesized polypeptide, and there are 57 polypeptides synthesized in all. Each polypeptide is coupled with carrier proteins KLH and BSA respectively (reference: Darwish IA, Alzoman NZ, Abuhejail RM, El-Samani TE. Chem Cent J 2012; 6:125), wherein the BSA-coupled polypeptide is used for serum antibody titer determination of Balb/c mice after the completion of immunizing, and the KLH-coupled polypeptide antigen is mixed with an adjuvant in equal volume, after emulsified fully, which is used to immunize Balb/c mice (8- to 12-week-old) via subcutaneous injection at multiple sites on neck and back for 3 times on day 1, day 15 and day 29 respectively. Freund's complete adjuvant is used for the first time, and Freund's incomplete adjuvant is used for the second and third time. Each mouse is injected with 500 µl of emulsified antigen each time, and the antigen dosage is 50 µg. After the completion of immunizing, the antibody titer in serum is detected by indirect ELISA method, and the results show that the corresponding polyclonal antibodies (antiserum) of 46 polypeptides are obtained and no corresponding antibodies are produced for 11 polypeptides.

### 1.2 Preparation ofHCVpp

The HCVpp is obtained by the cotransfection of 293T cells with HCV envelope protein E1E2 expression plasmid together with HIV gag/pol (pLP1), HIV rev (pLP2) and pLenti6 plasmid encoding luciferase. The HCV envelope protein E1E2 expression plasmid is constructed by cloning the HCV envelope protein E1E2 coding sequence into vector pCR3.1, and after the transfection of the 293T cells, HCV envelope protein E1E2 can be expressed. The DNA coding sequences of the envelope protein E1E2 of 7 different geno-types/subtypes of HCVused in the present invention are derived from infected subjects with different genotypes of HCV, and such DNA coding sequences are respectively cloned into vector pCR3.1 (Invitrogen) to construct the HCV envelope protein E1E2 expression plasmids. Please refer to Lavrillette D, Tarr AW, Voisset C, Donot P, Bartosch B, Bain C, Patel AH, Dubuisson J, Ball JK, Cosset FL. Hepatology 2005;41:265-274 for specific method. The HIV gag/pol (pLP1), HIV rev (pLP2) and pLenti6 plasmid coding luciferase are purchased from Invitrogen Company. The preparation process of the HCVpp comprises: cotransfecting 293T cells with 7 different geno-types/subtypes (genotypes 1a, 1b, 2a, 3a, 4a, 5 and 6) of HCV envelope protein E1E2 expression plasmids, together with HIV gag/pol (pLP1 ), HIV rev (pLP2) and pLenti6 plasmid encoding luciferase, placing in a cell incubator at 37°C after transfection, incubating for 48 h, and collecting cell culture supernatant containing HCVpp to obtain 7 different geno/subtypes of HCVpp (reference: Tong Y, Zhu Y, Xia X, Liu Y, Feng Y, Hua X, Chen Z, Ding H, Gao L, Wang Y, Feitelson MA, Zhao P , Qi ZT . J Virol, 2011; 85:2793-2802). The supernatant is filtered through a 0.45 µm filter, and concentrated by a factor of 20 through a 100K ultrafiltration concentration and centrifugation tube from PALL, USA, for use in HCVpp neutralization experiment.

### 1.3 HCVpp neutralization experiment and detection of luciferase activity

Huh7.5 cells are pre-inoculated into a 96-well plate containing DMEM complete medium, with an inoculum density of 1×10⁴/well), and then incubated in a cell incubator at 37°C. On the next day, the supernatants containing HCVpp (20 µl/well) aremixed with different polyclonal antibodies and negative control serum at different dilution ratios (the dilution ratios of polyclonal antibody to negative control serum are 1:50, 1:100, 1:200, 1:400 and 1:800 respectively), and the HCVpp supernatant without additions of the polyclonal antibody and the negative serum is also used as control, followed by adding polybrene in a final concentration of 4 µg/mlwith a total volume of 100 µl/well (DMEM complete medium is used to complement it to 100 µl in the case of not reaching100 µl), incubating at 37°C for 1 h, and adding into the 96-well plate and preparing 3 parallel wells for each dilution concentration. After incubating at 37°C for 5 h, the cell culture supernatant is discarded and replaced with fresh DMEM complete medium for continuous incubation at 37°C for 72 h. The detection is performed by using luciferase assay system (Promega), the method comprises discarding the cell culture supernatant, washing with PBS once, adding cell lysate (20 µl/well), incubating for 10 min, adding substrate luciferin (100 µl/well), placing into a fluorescence detector, and reading values.

### 1.4 Determination of neutralizing polyclonal antibody and protective B-cell epitope peptide

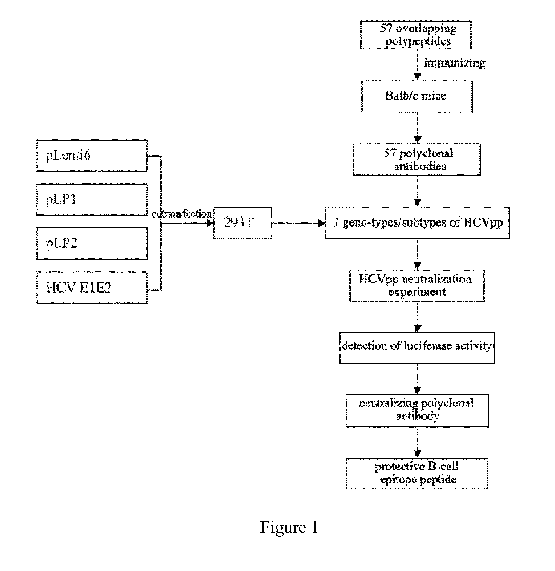

The polyclonal antibody, of which the value of luciferase activity is reduced by 50% or more after addition thereof compared to that of the control without additions of the polyclonal antibody and negative control serum, are considered to have neutralization activity. When the dilution ratio is 1:50, the corresponding antibody of PUHI26 can reduce the luciferase activity of HCVpp genosubtype 1a infected cell by about 71.3%, HCVpp genosubtype 1b infected cell by about 86.2%, HCVpp genosubtype 3a infected cell by about 63.5%, HCVpp genosubtype 4a infected cell by about 91.8%, HCVpp genotype 5 infected cell by about 53.9%, and HCVpp genotype 6 infected cell by about 54.9%. It can be determinated that the polyclonal antibody is a broadly neutralizing polyclonal antibody, and the epitope peptide identified by the polyclonal antibody, i.e. PUHI26 (the amino acid composition is THKFNSSGCPERMASCRPID), is a protective B-cell epitope peptide. The flow chart of the process for screening HCV envelope protein protective B-cell epitope peptide is shown in Figure 1.

### Example 2 Preparation and titer determination of corresponding antibodies of protective B-cell epitope peptide at 444-463 (PUHI26)

### 1.1 Preparation of corresponding antibodies of protective B-cell epitope peptide at 444-463 (PUHI26)

The artificially synthesized hepatitis C virus B-cell epitope peptide PUHI26 is coupled with KLH and BSA respectively (reference: Darwish IA, Alzoman NZ, Abuhejail RM, El-Samani TE. Chem Cent J 2012; 6:125), wherein the BSA-coupled epitope peptide is used for serum antibody titer determination of Balb/c mice after the completion of immunizing, the KLH-coupled epitope peptide antigen is mixed with an adjuvant in equal volume, and after emulsified fully, is used to immunize Balb/c mice (8- to 12-week-old) via subcutaneous injection at multiple sites on neck and back for 3 times on day 1, day 15 and day 29 respectively. Freund's complete adjuvant is used for the first time, and Freund's incomplete adjuvant is used for the second and third time. Each mouse is injected with 500 µl of emulsified antigen each time, and the antigen dosage is 50 µg.

### 1.2 Titer determination of corresponding antibodies of protective B-cell epitope peptide at 444-463 (PUHI26)

After the completion of immunizing, the serum antibody titer is detected by indirect ELISA method. The specific method comprises: coating the BSA-coupled corresponding epitope peptide antigen in a 96-well plate, blocking with 1×PBS containing 10% goat serum (200 µl/well), and incubating at 37°C for 1 h; washing with PBS for 3 times, adding the polyclonal antibodies (antiserum) of different dilution ratios of 1:1,000, 1:2,000, 1:4,000, 1:8,000, 1:16,000, 1:32,000, 1:64,000 and 1:128,000 respectively, and incubating at 37°C for 2 h; washing with PBST (1×PBS containing 0.05% Tween 20) for 4 times, and adding horse radish peroxidase-coupled anti-mouse IgG antibody, incubating at 37°C for 1 h; and washing with PBST for 4 times, adding substrate, incubating at room temperature for 30 minutes, ending the reaction with 2N HCl, and detecting the OD₄₉₀ value. 3 parallel wells are provided for each dilution concentration of the polyclonal antibody. A graph is drawn by taking dilution ratios and OD values as horizontal and vertical coordinates respectively. If the linear relationship is good, the OD value of polyclonal antibody is ≥0.25, and the ratio of the OD value of polyclonal antibody to that of negative control is ≥2 at the dilution ratio of 1:128,000 it can be indicated that the polyclonal antibody has good titer and can be used for HCVpp neutralization experiment.

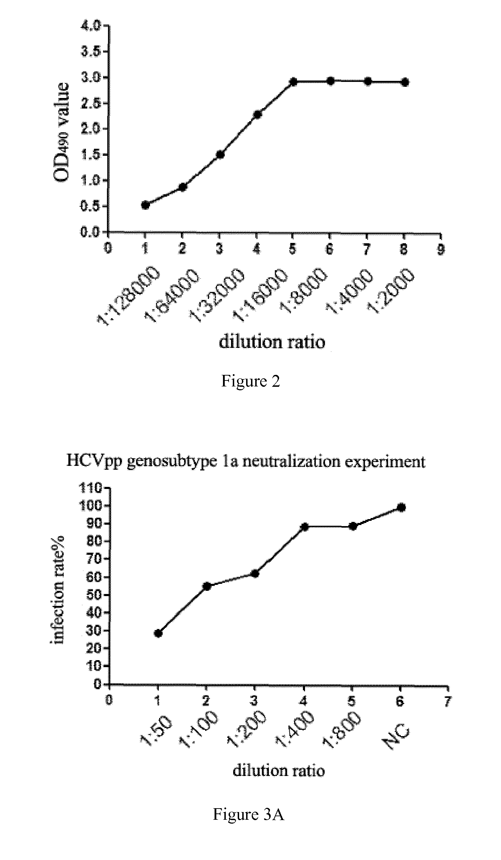

The titer determination results of corresponding antibodies of the protective B-cell epitope peptide at 444-463 (PUHI26) are shown in Figure 2.

### Example 3 Detection of neutralization of corresponding antibodies of B-cell epitope peptide at 444-463 (PUHI26) against HCVpp geno-types/subtypes 1a, 1b, 3a, 4a, 5 and 6 infected Huh7.5 cells

The polyclonal antibodies obtained are subjected to HCVpp neutralization experiment. The method comprises pre-inoculating Huh7.5 cells into a 96-well plate containing DMEM complete medium with an inoculum density of 1×10⁴/well, and incubating in a cell incubator at 37°C. On the next day, the supernatant containing HCVpp (20 µl/well) is mixed with different polyclonal antibodies and negative control serum at different dilution ratios (the dilution ratios of polyclonal antibody to negative control serum are 1:50, 1:100, 1:200, 1:400 and 1:800 respectively, and the HCVpp supernatant without addition of the polyclonal antibody and the negative serum is also used as control), followed by adding polybrene in a final concentration of 4 µg/ml, with a total volume of 100 µl/well (DMEM complete medium is used to complement it to 100 µl in the case of not reaching 100 µl), incubating at 37°C for 1 h, and adding into the 96-well plate, with 3 parallel wells for each dilution concentration. After incubating at 37°C for 5 h, the cell culture supernatant is discarded and replaced with fresh DMEM complete medium for continuous incubation at 37°C for 72 h. The detection is performed by using luciferase assay system (Promega), and the method comprises discarding the cell culture supernatant, washing with PBS once, adding cell lysate (20 µl/well), incubating for 10 min, adding substrate luciferin (100 µl/well), placing into a fluorescence detector and reading values. The statistic analysis is applied to the detection results, and shows that, compared to the control without addition of the polyclonal antibody and negative control serum, the luciferase activity value is reduced by 50% or more after adding the polyclonal antibody, indicating that the polyclonal antibody has neutralization activity. Accordingly, further assays are conducted to verify whether the antibody has neutralization activity against some or several geno-type(s)/subtype(s) of HCVpp.

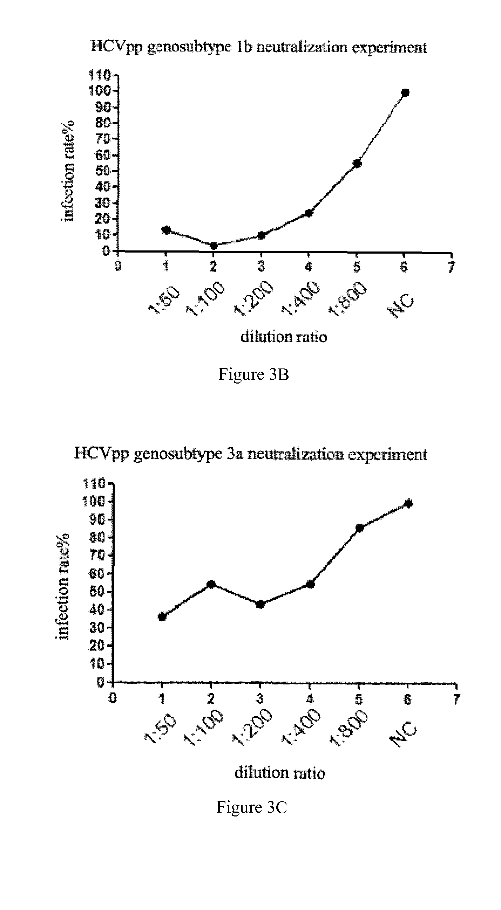

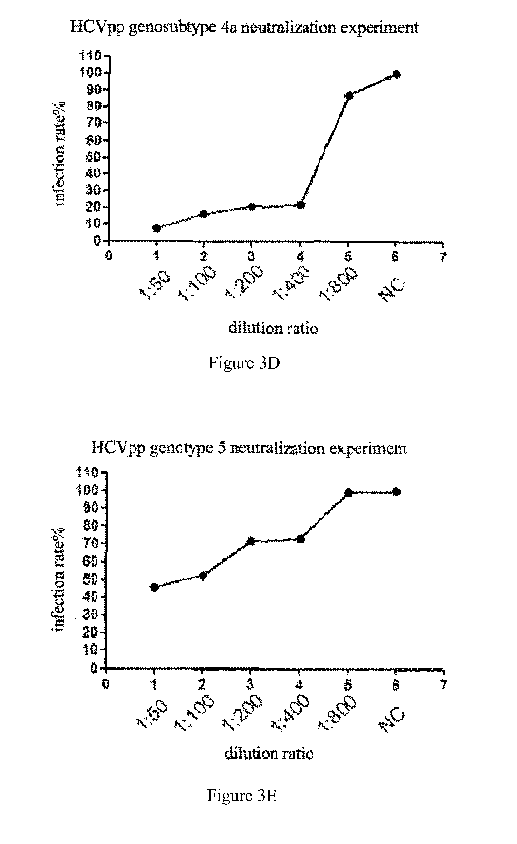

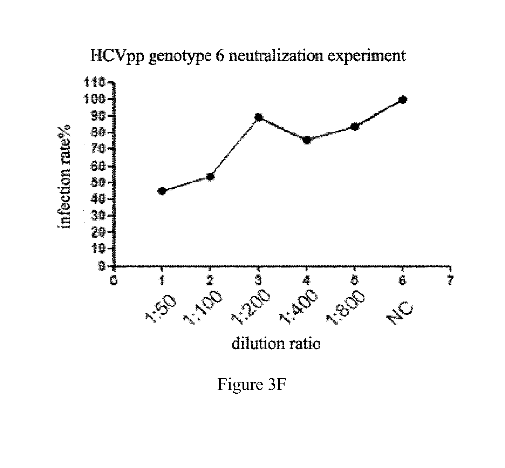

The results of neutralizationexperiments of corresponding antibodies of the protective B-cell epitope peptide at 444-463 (PUHI26) against HCVpp geno-types/subtypes 1a, 1b, 3a, 4a, 5 and 6 are shown in Figures 3A-3F.

While this invention has been specifically described with reference to the aforementioned general descriptions and specific embodiments, it is apparent to a person skilled in the art to make some modifications or improvements based on the present invention. Therefore, such modifications or improvements without departing from the spirit of the present invention fall within the scope of the present invention.

### Industrial practicality

The present invention adopts overlapping peptide method to obtain a novelprotective linear B-cell epitope peptide of hepatitis C virus envelope protein, where the amino acid sequence thereof is THKFNSSGCPERMASCRPID. It provides a protective antibody capable of neutralizing hepatitis C virus HCVpp geno-types/subtypes 1a, 1b, 3a, 4a, 5 and 6. The determination of the protective B-cell epitope peptide provides a new solution for the development of therapeutic antibody and prophylactic vaccine for HCV.

### References:

Ndongo N, Berthillon P, Pradat P, Vieux C, Bordes 1, Berby F, Maynard M, et al. Association of Anti-E1E2 Antibodies with Spontaneous Recovery or Sustained Viral Response to Therapy in Patients Infected with Hepatitis C Virus. Hepatology 2010; 52: 1531-1542.
Tong Y, Zhu Y, Xia X, Liu Y, Feng Y, Hua X, Chen Z, et al. Tupaia CD81, SR-BI, claudin-1, and occludin support hepatitis C virus infection. J Virol 2011; 85: 2793-2802.
Tarr AW, Urbanowicz RA, Jayaraj D, Brown RJ, McKeating JA, Irving WL, Ball JK. Naturally occurring antibodies that recognize linear epitopes in the amino terminus of the hepatitis C virus E2 protein confer noninterfering, additive neutralization. J Virol 2012; 86:2739-2749.
Darwish IA, Alzoman NZ, Abuhejail RM, El-Samani TE. Synthesis of hapten and preparation of specific polyclonal antibody with high affinity for lenalidomide, the potent drug for treatment of multiple myeloma. Chem Cent J 2012; 6:125.
Lavillette D, Tarr AW, Voisset C, Donot P, Bartosch B, Bain C, Patel AH, et al. Characterization of host-range and cell entry properties of the major genotypes and subtypes of hepatitis C virus. Hepatology 2005; 41:265-274.

## Claims

1. Hepatitis C virus B-cell epitope peptide PUHI26, wherein the amino acid composition thereof is THKFNSSGCPERMASCRPID.

2. Use of the hepatitis C virus B-cell epitope peptide PUH126 according to Claim 1 in the preparation of anti-HCV drugs.

3. The use according to Claim 2, wherein a polyclonal antibody is prepared by immunizing experimental animals using the epitope peptide PUHI26 as immunogen together with an adjuvant.

4. The use according to Claim 2, wherein a humanized monoclonal antibody identifying PUHI26 epitope peptide antigen is prepared by immunizing experimental animals using the PUHI26 epitope peptide as immunogen together with an adjuvant to, and adopting hybridoma technology and recombinant DNA technology.

5. A neutralizing antibody geno-types/subtypes 1a, 1b, 3a, 4a, 5 and 6, being a polyclonal antibody prepared by immunizing experimental animals using hepatitis C virus B-cell epitope peptide PUHI26 according to Claim 1 as immunogen together with adjuvant.

6. The neutralizing antibody according to Claim 5, wherein it is prepared by coupling the epitope peptide PUHI26 with a carrier protein KLH, mixing KLH-coupled epitope peptide antigen with the adjuvant in equal volume, after emulsified, immunizing Balb/c mice via subcutaneous injection for 3 times, and after the completion of immunizing, collecting the antibody in serum.

7. The neutralizing antibody according to Claim 6, wherein it is prepared by coupling the epitope peptide PUHI26 with the carrier protein KLH, mixing the KLH-coupled epitope peptide antigen and the adjuvant in equal volume, after completely emulsified, immunizing 8- to 12-week-old Balb/c mice via subcutaneous injectionat multiple sites on neck and back for 3 times on day 1, day 15 and day 29 respectively, wherein Freund's complete adjuvant is used for the first time, Freund's incomplete adjuvant is used for the second and third time, each mouse is injected with 500 µl of emulsified antigen each time, and the antigen dosage is 50µg, and collecting the antibody in serum after the completion of immunizing .


SEQUENCE LISTING

<110>  Peking University People's Hospital

<120>  Hepatitis C virus B-cell epitope peptide PUH126 and application
       thereof

<130>  KHP143110671

<140>  PCT/CN2014/072645
<141>  2014-02-27

<160>  1

<170>  PatentIn version 3.5

<210>  1
<211>  20
<212>  PRT
<213>  Hepatitis C virus

<400>  1

Thr His Lys Phe Asn Ser Ser Gly Cys Pro Glu Arg Met Ala Ser Cys
1               5                   10                  15


Arg Pro Ile Asp
            20